**Europäisches Patentamt**

**European Patent Office**

**Office européen des brevets**

(11) Veröffentlichungsnummer: **0 285 735**
**A1**

# (12) EUROPÄISCHE PATENTANMELDUNG

(21) Anmeldenummer: 87730036.8

(22) Anmeldetag: 08.04.87

(51) Int. Cl.⁴: **A61F 2/36**

(43) Veröffentlichungstag der Anmeldung:
**12.10.88 Patentblatt 88/41**

(84) Benannte Vertragsstaaten:
**CH ES FR GB IT LI**

(71) Anmelder: **MECRON MEDIZINISCHE**
**PRODUKTE GMBH**
**Nunsdorfer Ring 23-27**
**D-1000 Berlin 48(DE)**

(72) Erfinder: **Kranz, Curt**
**Kufsteiner Strasse 12**
**D-1000 Berlin 62(DE)**
Erfinder: **Anapliotis, Emmanuel**
**Kiebitzweg 5**
**D-1000 Berlin 33(DE)**

(74) Vertreter: **Christiansen, Henning, Dipl.-Ing.**
**CHRISTIANSEN & NINNEMANN**
**Dietrich-Schäfer-Weg 21**
**D-1000 Berlin 41(DE)**

(54) Verfahren zur Herstellung eines nicht spiegelsymmetrischen Schaftes einer Hüftgelenkprothese.

(57) Verfahren zur Herstellung eines nicht spiegelsymmetrischen Schaftes (I) einer implantierbaren femoralen Hüftgelenkprothese, der in den Markraum eintreibbar ist und eine unsymmetrische Erhebung, insbesondere in Form von in Eintreibrichtung verlaufenden Rippen (6, 7) oder Rippenteilen, der entweder als sogenannter linksseitiger oder als rechtsseitiger Schaft für einen linken bzw. rechten Oberschenkel vorgesehen ist und Bereiche (2) aufweist, die sich beim linksseitigen und beim rechtsseitigen Schaft zur Deckung bringen lassen, also spiegelsymmetrisch sind, wobei von einem bevorzugt spiegelsymmetrischen Halbfertigfabrikat ausgegangen wird, bei dem die genannten spiegelsymmetrischen Bereiche (2) als im wesentlichen nicht mehr spanabhebend nachzubearbeitende Fertigbereiche ausgebildet sind, während andere Bereiche (6, 7) durch spanabhebende Verformung soweit abgetragen werden, bis der Schaft die Form des fertigen entweder linksseitigen oder rechtsseitigen Schaftes erreicht hat, wobei gegebenenfalls die Rippen nachträglich durch Fräsen erzeugt werden sowie entsprechendes Halbfabrikat.

## Verfahren zur Herstellung eines nicht spiegelsymmetrischen Schaftes einer Hüftgelenkprothese

Verfahren zur Herstellung eines nicht spiegelsymmetrischen, implantierbaren Schaftes einer Hüftgelenkprothese, nach diesem Verfahren hergestellter Schaft und zugehöriges Halbfertigfabrikat.

Die Erfindung betrifft ein Verfahren zur Herstellung eines nicht spiegelsymmetrischen, implantierbaren Schaftes einer femoralen Hüftgelenkprothese, der in eine Markhöhle eintreibbar ist und insbesondere Rippen oder Rippenteile aufweist, die in Eintreibrichtung verlaufen, und der entweder als sogenannter linksseitiger oder als rechtsseitiger Schaft für einen linken bzw. rechten Oberschaenkel geeignet ist und Bestandteile aufweist, die sich beim linksseitigen und beim rechtsseitigen Schaft zur Deckung bringen lassen, also spiegelsymmetrisch sind.

Außerdem betrifft die Erfindung einen Schaft einer nicht spiegelsymmetrischen, implantierbaren links-rechtsseitigen Hüftgelenkprothese, bei dem wenigstens einer von zwei nicht spiegelsymmetrischen Oberflächenteilen Rippen oder Rippenteile aufweist.

Ein derartiger Schaft ist aus der DE-OS 32 l6 539 bekannt. Er besitzt einen Kopfteil und ist zur zementfreien Verankerung in einem zum Prothesenende hin sich erweiternden Röhrenknochen vorgesehen. In seinem kopfnahen Bereich ist er zur Anlage an der Corticalis des Knochens bemessen. Seine entsprechende Außenfläche wird von einer Mehrzahl von Rippen gebildet, die zwischen sich Raum zur Aufnahme von Knochengewebe einschließen. Dies ermöglicht zum einen eine den Eigenschaften der Knochensubstanz angepaßte, biegeweiche Ausführung des dicken, kopfnahen Schaftteils und - bei Gestaltung der Rippen entsprechend der Europäischen Patentanmeldung l4l 022 eine - ausreichende Primär-Stabilität der Prothese nach der Implantation. Andererseits ermöglichen die Rippen zufolge der Angaben in der DE-OS 32 l6 539 die unaufwendige Bereitstellung und gegebenenfalls individuelle Anpassung einer Vielzahl von Maß-und Formvariationen ausgehend von einer geringen Zahl ursprünglicher Formen durch Nachbearbeitung der Außenoberflächen.

Der in der vorgenannten Schrift beschriebene und dargestellte Schaft ist aber bezüglich seiner geschwungenen Form und der Anordnung der Rippen so gestaltet, daß er nur für einen der beiden menschlichen Oberschenkel geeignet ist. Für den anderen Oberschenkel müßte ein bezüglich der Körpersymmetrieebene spiegelbildlich gestalteter Schaft gesondert hergestellt werden.

Der im kennzeichnenden Teil des Anspruchs l angegebenen Erfindung liegt die Aufgabe zugrunde, ein Herstellungsverfahren der eingangs genannten Gattung und einen Schaft anzugeben, der mit geringem Aufwand die wahlweise Herstellung eines linksseitig oder rechtsseitig verwendbaren Schaftes ermöglicht. Auf diese Weise hergestellte Schäfte für die eine oder andere Körperseite sollen sich also durch geringen Anpassungsaufwand für die beiden Grundformen (links und rechts) herstellen lassen.

Die nicht zur Deckung bringbaren Bereiche, die beim Fertigprodukt also die nicht spiegelsymmetrischen Bestandteile verkörpern, sind beim Vorfabrikat als Halbfertigbereiche ausgebildet und bestehen aus Vollmaterial oder auch aus bereits vorgefertigten Rippen oder Rippenteilen. Während des Herstellungsverfahrens werden nun solche Halbfertigbereiche durch spanabhebende Verformung, z.B. Fräsen, soweit abgetragen, bis der Schaft die Form des späteren unsymmetrischen entweder linksseitigen oder rechtsseitigen Schaftes erreicht hat.

Ein auf diese Weise hergestellter Schaft zeichnet sich nach der Erfindung dadurch aus, daß von den beiden nicht spiegelsymmetrischen Oberflächenteilen, von denen einer Rippen oder Rippenteile aufweist, der andere Oberflächenteil spanabhebend gearbeitet ist und dadurch weniger als der andere nicht spiegelsymmetrische Oberflächenteil bzw gar keine Rippen aufweist.

Durch das erfindungsgemäße Verfahren ist es weiterhin in vorteilhafter Weise möglich, unterschiedliche Formvarianten - ausgehend von identischen Rohlingen - sowohl für Links-als auch für Rechtsausführung zu erzeugen. Das ist insbesondere deswegen günstig, weil wegen der verschiedenen erforderlichen Prothesengrößen ohnehin viele verschiedene Formvarianten als Rohlinge auf Lager gehalten werden müssen.

Die Beseitigung von massivem Material zum Einebnen einer Oberfläche erweist sich in der Regel in der Fertigung als relativ schnell ausführbar, da keine besonders gesteuerten Spezialfräser Anwendung finden müssen. Die endgültige Rippenstruktur wird dann in einem abschließenden Nachbearbeitungsvorgang erzeugt.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Zeichnung näher dargestellt. Es zeigen:

Figuren l bis 4 jeweils paarweise zwei Ansichten zweier Schäfte, von denen jeweils einer für eine der beiden Körperseiten bestimmt ist. Es handelt sich um fertige Schäfte,

Figur 4a das Ausführungsbeispiel gemäß Figur 4 im Schnitt sowie

Figur 5 eine den Figuren 2 und 3 entsprechende Ansicht eines Halbfertigfabrikates, das zur Herstellung der Schäfte nach den Figuren I, 2 bzw. 3, 4 geeignet ist.

Die Schäfte I, die mit ihrem distalen Ende 2 voran in eine Markhöhle eintreibbar sind, weisen an ihrem proximalen Ende 3 einen Konus 4 zur Aufnahme einer Gelenkkugel 5 auf.

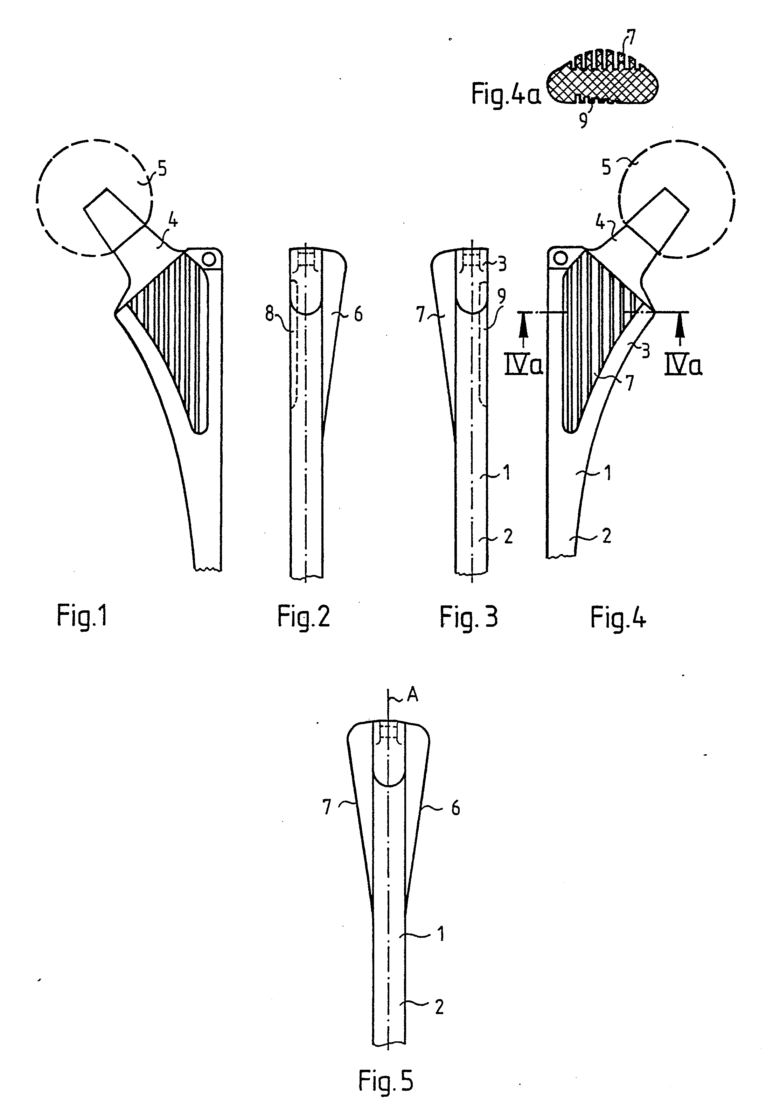

Es ist ersichtlich, daß die distalen Schaftenden jeweils spiegelsymmetrisch zu einer Spiegelebene ausgebildewt sind, die bei den Figuren 2, 3, 5 parallel zu einer Ebene A verläuft, die senkrecht auf der Zeichenebene steht. Beim Halbfertigfabrikat nach 4 und 5 sind diese spiegelsymmetrischen Bestandteile als nicht mehr zu bearbeitende Fertigbereiche ausgebildet.

Daneben gibt es andere Bestandteile, nämlich die die späteren Rippen 6 und 7 aufnehmenden Volumenbereiche am Halbfertigfabrikat nach Figur 5, die an diesem zwar auch spiegelsymmetrisch angeordnet sein können, denen aber beim fertigen Schaft nach den Figuren I bis 4 keine Bestandteile entsprechen, die dort spiegelsymmetrisch sind, die sich also beim linksseitigen und beim rechtsseitigen Schaft zur Deckung bringen lassen. Der Schaft nach Figur 2 weist nämlich lediglich die Rippen 6 und der Schaft nach Figur 3 lediglich die Rippen 7 auf, während die jeweils die anderen Rippen enthaltenden Volumenbereiche während der Ausübung des Herstellungsverfahrens entfernt worden sind (oder auch nur teilweise entfernt worden sein können), und zwar durch spanabhebende Verformung, die soweit durchgeführt worden ist, bis die in den Figuren I bis 4 gezeigte Schaftform erreicht war. Die Rippenstruktur wird durch eine Nachbearbeitung mittels eines Fräsers erzeugt. Auf diese Weise ist für unterschiedlichste Ausführungen der Prothese einer Größe nur eine einzige Rohlingform erforderlich.

Dieser fertige Schaft weist also außer seinen spiegelsymmetrischen Oberflächenteilen noch zwei nicht-spiegelsymmetrische Oberflächenteile auf, von denen einer durch die Rippen 6 bzw. 7 (Figur 2 bzw. 3) gebildet ist, während der andere, jeweils gegenüberliegende, nicht spiegelsymmemetrische Oberflächenteil 8 bzw. 9 Spuren einer spanabhebenden Bearbeitung aufweist, so daß an den Oberflächenteilen 8 bzw. 9 später keine Rippen mehr oder weniger hohe Rippen als an den Oberflächenteilen 6 und 7 erzeugt werden können.

Die den erhaben ausgebildeten Rippenbereichen gegenüberliegenden rippenförmigen Bereiche sind bei einer in Figur 4a im Schnitt dargestellten bevorzugten Ausführungsform vertieft ausgebildet. Nach dem Entfernen des erhabenen Volumenmaterials durch eine planierenden Obverflächenbearbeitung werden hier also zusätzlich mittels eines bevorzugt programmgesteuerten Fräsers parallele nutförmige Vertiefungen erzeugt.

Die Erfindung ermöglicht es, bei der Herstellung linksseitiger und rechtsseitiger Schäfte von einem einheitlichen Halbfertigfabrikat nach Figur 5 auszugehen. Des weiteren brauchen bei der Weiterverarbeitung zum fertigen Schaft brauchen nur noch erhabene Volumen Rippen oder Rippenteile spanabhebend entfernt zu werden. Dies ist insbesondere dann besonders leicht möglich, wenn beim Halbfertigfabrikat all diejenigen Volumenteile aus Massivmaterial oder als Rippen oder Rippenteile ausgebildet sind, die jeweils nur bei einem der beiden möglichen Schäfte (linksseitiger Schaft bzw. rechtseitiger Schaft) vorkommen, aber nicht bei beiden gleichzeitig, wie es bei den spiegelsymmetrischen Bestandteilen der fertigen Schäfte der Fall ist.

Die Volumenteile oder Oberflächenteile (beide Möglichkeiten entsprechen einander) 6 und 7 in Figur 5, also am Halbfertigfabrikat, stimmen genau mit denjenigen Bereichen 6 und 7 der fertigen Schäfte nach den Figuren I bis 4 überein, die sich nicht zur Deckung bringen lassen, wenn man ansonsten einen links-und einen rechtseitigen Schaft zur Deckung zu bringen. Die Oberflächenbereiche 6 und 7 mit Figur 5 umschließen die in den Figuren 2 und 3 nicht zur Deckung bringbaren Bereiche 6 und 7.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch machen.

## Ansprüche

I. Verfahren zur Herstellung eines nicht spiegelsymmetrischen Schaftes (I) einer implantierbaren femoralen Hüftgelenkprothese, der in den Markraum eintreibbar ist und eine unsymmetrische Erhebung, insbesondere in Form von in Eintreibrichtung verlaufenden Rippen (6, 7) oder Rippenteilen, der entweder als sogenannter linksseitiger oder als rechtsseitiger Schaft für einen linken bzw. rechten Oberschenkel vorgesehen ist und Bereiche (2) aufweist, die sich beim linksseitigen und beim rechtsseitigen Schaft zur Deckung bringen lassen, also spiegelsymmetrisch sind,

**dadurch gekennzeichnet,**

daß von einem bevorzugt spiegelsymmetrischen Halbfertigfabrikat (4 und 5) ausgegangen wird, bei dem die genannten spiegelsymmetrischen Bereiche (2) als nicht mehr spanabhebend zu bearbeitende Fertigbereiche ausgebildet sind, während andere Bereiche (6, 7 in Figur 5) eine Gestalt aufweisen, welche die nicht zur Deckung bringbaren Bereiche sowohl des links-als auch des rechtsseitigen Schaftes (Figuren I bis 4) einschließen, wobei diese Bereiche (6, 7 in Figur 5) als Halbfertigbereiche ausgebildet sind und aus Voll-oder vorstrukturiertem Material bestehen, und daß dieses Material (6, 7 in Figur 5) an den Halbfertigbereichen durch spanabhebende Verformung soweit abgetragen wird, bis der Schaft die Form des fertigen entweder linksseitigen oder rechtsseitigen Schaftes (Figuren I bis 4) erreicht hat, wobei gegebenenfalls die Rippen nachträglich durch Fräsen erzeugt werden.

2. Nicht spiegelsymmetrischer Schaft einer implantierbaren links-bzw. rechtsseitigen Hüftgelenkprothese bei dem wenigstens einer von zwei nicht-spiegelsymmetrischen Oberflächenteilen Rippen (6, 7 in den Figuren 2, 3) oder Rippenteile aufweist, **dadurch gekennzeichnet,** daß der Oberflächenbereiche (8, 9) zur Erzeugung in die Oberfläche versenkter Rippenstrukturen spanabhebend bearbeitet werden.

3. Halbfertigfabrikat für die Herstellung eines linksseitigen oder rechtsseitigen Schaftes nach einem der vorangehenden Ansprüche, **gekennzeichnet durch** die volumenmäßige Überlagerung mindestens aller sowohl bei dem linkseitigen als auch bei dem rechtsseitigen Schaft (Figuren I bis 4) vorkommenden Bereiche.

Fig.4a

Fig.1  Fig.2  Fig. 3  Fig.4

Fig. 5

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int. Cl.4) |
|---|---|---|---|
| A | EP-A-0 182 176 (WALDEMAR LINK)<br>* Anspruch; Figuren 1-5 *<br>--- | 1 | A 61 F 2/36 |
| A | WO-A-8 503 426 (JOINT MEDICAL PRODUCTS CORP.)<br>* Figuren 1-6; Seite 7, Zeile 22 - Seite 8, Zeile 2 *<br>--- | 1 | |
| D,A | DE-A-3 216 539 (WALDEMAR LINK)<br>* Figuren 1-5; Seite 9, Zeilen 9-20 *<br>--- | 1 | |
| D,A | EP-A-0 141 022 (GEBR. SULZER AG)<br>* Figur 2 *<br>----- | 1 | |

**RECHERCHIERTE SACHGEBIETE (Int. Cl.4)**

A 61 F 2/00

Der vorliegende Recherchenbericht wurde für alle Patentansprüche erstellt

| Recherchenort | Abschlußdatum der Recherche | Prüfer |
|---|---|---|
| BERLIN | 04-11-1987 | KANAL P K |

EPO FORM 1503 03.82 (P0403)

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angefzhrtes Dokument
.....................................................................................
& : Mitglied der gleichen Patentfamilie, übereinstimmendes Dokument